# EUROPÄISCHE PATENTANMELDUNG

(11) **EP 2 311 525 A1**
(43) Veröffentlichungstag der Anmeldung: **20.04.2011**
(21) Anmeldenummer: 10013524.3
(22) Anmeldetag: 12.10.2010
(51) Int. Cl.: A61N 5/01, A61N 5/06

(54) **Vorrichtung zur Durchführung einer selektiven medizinischen Hyperthermie**

(30) Priorität: 13.10.2009 DE 102009049224
(71) Anmelder: Bolt, Christoph, 9430 St. Margrethen (CH)
(72) Erfinder: Bolt, Christoph, 9430 St. Margrethen (CH)
(74) Vertreter: Riebling, Peter

(57) **Zusammenfassung**

Verfahren zur Durchführung einer selektiven, medizinischen Hyperthermie mittels elektromagnetischer Wellen zur Behandlung des menschlichen oder tierischen Körpers, gekennzeichnet durch folgende Verfahrensschritte
e) Lagern der zu behandelnden Person (12) auf einer Liege (1) oder Fixierung eines Tieres auf einer Behandlungsebene
f) selektive Bestrahlung der zu behandelnden Person (12) oder des Tieres mit einer oder mehreren IR-Strahlungsquellen (7), die in einem mindestens in Richtung der Längsachse (13) der Person oder des Tieres verfahrbar angetriebenen Schlitten (4) angeordnet sind
g) Bestrahlung angewählter Bereiche des menschlichen oder tierischen Körpers mit Tiefeneinwirkung der IR-Strahlung in das menschliche Gewebe
h) Erzeugung einer lokalen Hyperthermie in den ausgewählten Behandlungsbereichen (14)

## Beschreibung

Gegenstand der Erfindung ist ein Verfahren zur Durchführung einer selektiven, medizinischen Hyperthermie mittels elektromagnetischer Wellen zur Behandlung des menschlichen oder tierischen Körpers zur Erhöhung des Wohlbefindens.

Eine solche Vorrichtung ist beispielsweise mit dem Gegenstand der DE 2423399 A1 bekannt geworden. Bei dieser Druckschrift wird das menschliche Gewebe mit einem Hohlspiegel bestrahlt, in dessen Brennpunkt eine elektromagnetische Strahlungsquelle angeordnet ist, welche einen stark fokussierten, elektromagnetischen Strahl auf das menschliche Gewebe richtet. Damit ist allerdings der Nachteil verbunden, dass nur eine stark lokale Behandlung des menschlichen oder tierischen Körpers möglich ist, die mit der Gefahr verbunden ist, dass örtlich lokale Schädigungen des Gewebes stattfinden. Eine solche Vorrichtung ist nicht geeignet, das menschliche Wohlbefinden zu erhöhen, indem schonend einzelne Bereiche des menschlichen oder tierischen Körpers einer gezielten Hyperthermie ausgesetzt werden, um beispielsweise die Durchblutung anzuregen, den Lymphfluss zu verbessern und dergleichen mehr. Gerade bei Sportlern ist es wichtig, gezielt aus bestimmten Körperbereichen die Milchsäure-Abbauprodukte nach sportlicher Höchstleistung abzutransportieren, was durch eine lokale Hyperthermie erfolgen kann.

Ein Gerät zur Behandlung der menschlichen Haut mit Einwirkung von Licht, Magnet, Ultraschall und dergleichen ist beispielsweise auch mit dem Gegenstand der DE 196 42 829 A1 bekannt geworden. Bei dieser Druckschrift besteht jedoch ebenfalls der Nachteil, dass mit einem Behandlungskopf auf der Haut aufgesetzt wird und dass damit kein kontinuierliches Fortschreiten des Behandlungskopfes in einem bestimmten Behandlungsbereich des menschlichen Körpers gewährleistet ist.

Der Erfindung liegt deshalb die Aufgabe zugrunde, ein Verfahren und eine Vorrichtung der eingangs genannten Art so weiterzubilden, dass bei Auswahl eines bestimmten lokalen Behandlungsbereiches des menschlichen oder tierischen Körpers eine günstige Tiefenwirkung der elektromagnetischen Strahlen auf das menschliche oder tierische Gewebe erzielt wird, ohne dass die Haut geschädigt wird.

Zur Lösung der gestellten Aufgabe ist das Verfahren durch die technische Lehre des Anspruches 1 gekennzeichnet.

Eine Vorrichtung zur Ausführung des Verfahrens ist nach der technischen Lehre des Anspruches 3 ausgebildet.

Wesentliches Merkmal der Erfindung ist, dass erfindungsgemäß ein oder mehrere entlang der zu behandelnden Haut bewegten IR-Strahlungsquellen verwendet werden, weil sich herausgestellt hat, dass mit solchen Strahlungsquellen hohe Strahlungsleistung und eine Eindringtiefe von bis zu 6 cm ins Gewebe erreicht werden kann.

Es wird mit einer relativ hohen Strahlungsleistung von z. B. 1200 Watt bis 2000 Watt Leistung der IR-Bestrahlungslampen gearbeitet. Würde die IR-Strahlungsquelle mit einer derartigen Strahlungsleistung auch nur mehr als 2 -5 Sekunden an der zu behandelnden Stelle stillstehen, kommt es zu unerwünschten Verbrennungen. Eine Verbrennung der Haut soll jedoch auf jeden Fall vermieden werden. Zu diesem Zweck ist erfindungsgemäß vorgesehen, dass die eine oder mehrere IR-Strahlungsquellen in einem Schlitten angeordnet sind, der mindestens in Richtung der Längsachse des menschlichen oder tierischen Körpers verschiebbar angetrieben ist. Die Bewegungsgeschwindigkeit und der Abstand sind abhängig von der Strahlungsleistung so gewählt, dass eine Schädigung des behandelten Gewebes ausgeschlossen ist.

Nach einer besonders bevorzugten Ausführungsform der vorliegenden Erfindung ist es vorgesehen, dass nach Auswahl eines bestimmten Handlungsbereiches (z. B. des menschlichen oder tierischen Unterschenkels oder Oberschenkels) der Schlitten mit einer Geschwindigkeit von etwa 2,5 bis 3 m pro Minute nur über diesen lokal ausgewählten Behandlungsbereich hinweg streicht und hierbei beispielsweise eine Behandlungszeit von 10 min bis 120 min vorgesehen werden kann.

Der Schlitten wird erfindungsgemäß oszillierend angetrieben, d. h. er überstreicht nur hin und her gehend das ausgewählte Behandlungsgebiet, wobei seine Geschwindigkeit und der Abstand der IR-Strahlungsquellen zum bestrahlten Gebiet so ausgewählt wird, dass eine Schädigung der menschlichen oder tierischen Haut auf jeden Fall vermieden wird. Dies kann durch eine Messung der Temperatur der Hautoberfläche während der Behandlung überprüft werden.

Es handelt sich also um ein Durchlaufverfahren, weil der Schlitten mit den dort angeordneten Strahlungsquellen das Behandlungsgebiet überstreicht und eine solche Geschwindigkeit und einen Abstand zum Behandlungsgebiet einnimmt, dass eine Schädigung des menschlichen oder tierischen Gewebes vermieden wird.

Versuche haben gezeigt, dass ein Abstand von etwa 40 bis 70 cm von der Oberfläche der Bestrahlungsquelle zur Oberfläche des Behandlungsgebietes günstig ist. Dieser Abstand ist in vertikaler Richtung einstellbar.

Die Einstellung kann hierbei entweder durch vertikale Einstellung am Schlittensystem für die Halterung des Schlittens, am Schlitten selbst oder an den Laufschienen des Schlittensystems vorgenommen werden.

Ebenso kann auch die Liege selbst höheneinstellbar ausgebildet sein, um den vertikalen Abstand zum Schlittensystem einstellbar zu gestalten.

In einer weiteren Ausführung der Erfindung kann es vorgesehen sein, dass der Schlitten mit den dort eingebauten Strahlungsquellen nicht nur in Richtung der Längsachse (X-Achse) über den menschlichen oder tierischen Körper streicht, sondern zusätzlich auch noch in Richtung der Querachse (Y-Achse) hierzu. Hier handelt es sich um Pendelbewegungen der IR-Strahlungsquelle in einer X-Y-Ebene parallel zur Oberfläche des Behandlungsgebietes. Damit sind oszillierende Halbkreis- oder Viertelkreis- oder Achtelkreis-Bewegungen über das Behandlungsgebiet gemeint.

Ebenso fallen darunter oszillierende, geradlinige Bewegungen in X- und Y-Richtung, wenn die Z-Achse die Normale auf der Körperoberfläche darstellt. Demzufolge können sich überlagernde oszillierende Verschiebebewegungen in Richtung der Längs- und Querachse vorgesehen sein.

In einer anderen Ausgestaltung der Erfindung kann es vorgesehen sein, dass der Schlitten nur in Richtung der Querachse (also senkrecht zur Längsachse) verschiebbar und oszillierend angetrieben ist.

In einer Weiterbildung der Erfindung kann es vorgesehen sein, die Strahlungsleistung der IR-Strahlungsquellen einstellbar zu gestalten, und die Einstellung kann auch in Abhängigkeit von der Verschiebegeschwindigkeit des Schlittens und dem sich gegebenenfalls ändernden Abstand zur Körperoberfläche automatisch eingestellt werden.

So kann es beispielsweise vorgesehen sein, dass die Strahlungsleistung an den Umkehrpunkten des Schlittens (Begrenzungen des Behandlungsbereiches) stark herabgesetzt wird, weil die IR-Strahlungsquellen an diesen Randbereichen in Vorwärts- und Rückwärtsrichtung kurz nacheinander das Behandlungsgebiet überstreichen und so lokal eine hohe Bestrahlungsleistung auf das menschliche Gewebe in diesem Bereich ausgeübt wird. Um eine Schädigung in diesen Umkehrbereichen zu vermeiden, kann es vorgesehen sein, dass automatisch die Bestrahlungsleistung an diesen Umkehrpunkten herabgesetzt wird.

Ebenso kann vorgesehen sein, die Verschiebegeschwindigkeit und/oder den Abstand der IR-Strahlungsquelle und/oder deren Strahlungsleistung in Abhängigkeit von der maximal zulässigen Hauttemperatur im Behandlungsgebiet zu regeln. Damit wird eine automatische Regelung vorgeschlagen, die zuverlässig eine durch Verbrennung bewirkte Hautschädigung ausschliesst.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, werden als erfindungswesentlich beansprucht, soweit sie einzeln oder in Kombination gegenüber dem Stand der Technik neu sind.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Draufsicht auf eine Vorrichtung nach der Erfindung

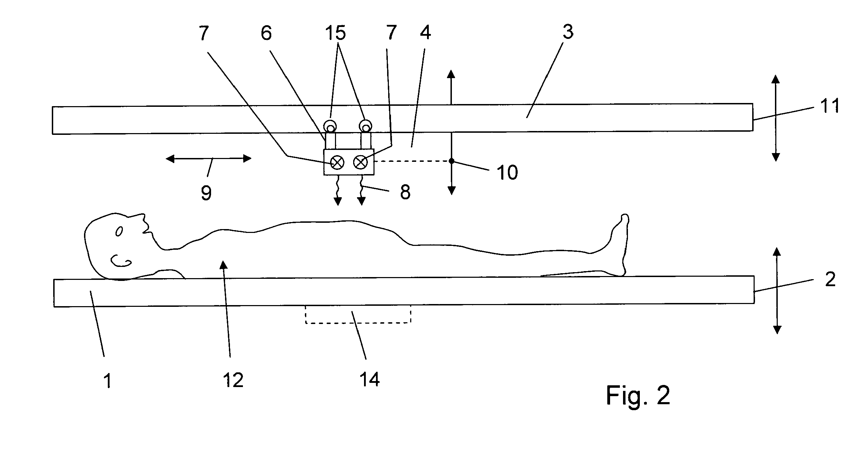

- Figur 2:: Seitenansicht der Vorrichtung nach Figur 1

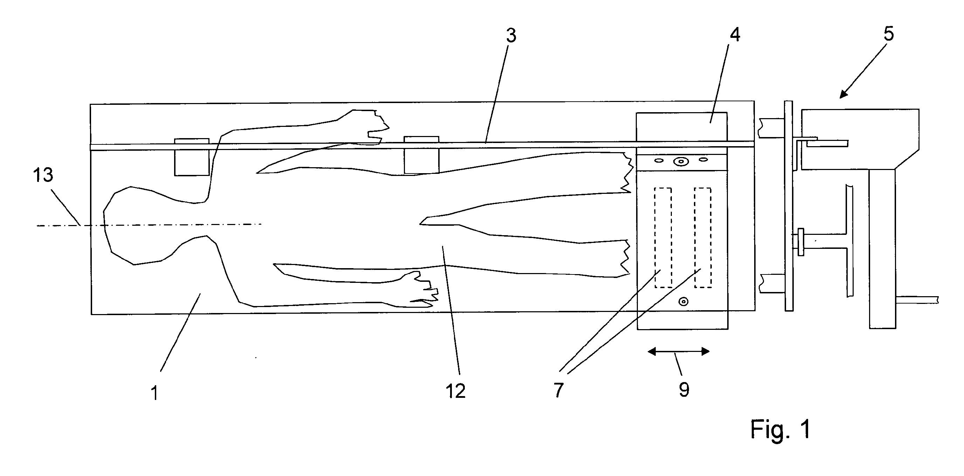

In Figur 1 ist eine Liege 1 dargestellt, auf der eine zu behandelnde Person 12 aufliegt. Die Längsachse der Liege ist mit 13 bezeichnet.

Oberhalb der Liege 1 ist eine Laufschiene 3 angeordnet, in der ein Schlitten 4 in den Pfeilrichtungen 9 verfahrbar angetrieben ist. Im Schlitten 4 sind ein oder mehrere IR-Strahlungsquellen 7 angeordnet, die eine gleichmäßige Bestrahlungsleistung auf den Behandlungsbereich 14 des menschlichen oder tierischen Körpers erbringen. Um eine schonende Behandlung des menschlichen oder tierischen Körpers zu gewährleisten, kann es vorgesehen sein, dass die Liege 1 in vertikaler Pfeilrichtung 2 einstellbar ausgebildet ist.

Ebenso kann es vorgesehen sein, dass die Laufschiene 3 in vertikaler Pfeilrichtung 11 einstellbar und feststellbar ausgebildet ist.

Die Strahlungsleistung der in dem Schlitten 4 angeordneten IR-Strahlungsquellen wird mit einer Steuerungseinheit 5 gesteuert, wobei zusätzlich noch die Strahlungsleistung im Schlitten 4 einstellbar ausgebildet sein kann.

In der Steuerungseinheit 5 ist der motorische Antrieb für den Schlitten 4 angeordnet, der beispielsweise über einen längs der Laufschiene 3 laufenden Zahnriemen in der Pfeilrichtung 9 oszillierend und verschiebbar angetrieben ist.

Nach Auswahl des Behandlungsbereiches 14 läuft somit der Schlitten 4 nur noch oszillierend über das Behandlungsgebiet 14, wobei er an den Rändern des Behandlungsbereiches umkehrt und wieder zurückläuft.

Der Schlitten 4 kann über eine Aufhängung 6 mit den Rollen 15 verbunden sein, die ihrerseits drehbar in der Laufschiene 3 aufgenommen sind. Die in dem Schlitten 4 aufgenommenen IR-Strahlungsquellen 7 bestrahlen somit den Behandlungsbereich 14 in Pfeilrichtung 8.

Es kann noch zusätzlich vorgesehen sein, dass der Schlitten 4 in den Pfeilrichtungen 10 vertikal einstellbar und feststellbar an der Laufschiene 3 gehalten ist. Um eine automatische Einstellung der Bestrahlungsleistung und/oder der Geschwindigkeit des Schlittens 4 zu gewährleisten, ist es in einer Weiterbildung der Erfindung vorgesehen, dass im Behandlungsbereich 14 mindestens ein Temperatursensor und/oder ein Bestrahlungssensor angeordnet sind, welche eine unzulässige hohe Temperatur und/oder Bestrahlungsleistung erfassen und dementsprechend die Lichtstärke der IR-Strahlungsquellen und/oder den Verschiebungsweg und/oder die Verschiebungsgeschwindigkeit des Schlittens 4 automatisch einstellen.

### Zeichnungslegende

- 1: Liege
- 2: Pfeilrichtung
- 3: Laufschiene
- 4: Schlitten
- 5: Steuerungseinheit
- 6: Aufhängung
- 7: IR-Strahlungsquelle
- 8: Pfeilrichtung
- 9: Pfeilrichtung
- 10: Pfeilrichtung
- 11: Pfeilrichtung
- 12: Person
- 13: Längsachse
- 14: Behandlungsbereich
- 15: Rollen

## Patentansprüche

1. Verfahren zur Durchführung einer selektiven, medizinischen Hyperthermie mittels elektromagnetischer Wellen zur Behandlung des menschlichen oder tierischen Körpers, **gekennzeichnet durch** folgende Verfahrensschritte
a) Lagern der zu behandelnden Person (12) auf einer Liege (1) oder Fixierung eines Tieres auf einer Behandlungsebene
b) selektive Bestrahlung der zu behandelnden Person (12) oder des Tieres mit einer oder mehreren IR-Strahlungsquellen (7), die in einem mindestens in Richtung der Längsachse (13) der Person oder des Tieres verfahrbar angetriebenen Schlitten (4) angeordnet sind
c) Bestrahlung angewählter Bereiche des menschlichen oder tierischen Körpers mit Tiefeneinwirkung der IR-Strahlung in das menschliche Gewebe
d) Erzeugung einer lokalen Hyperthermie in den ausgewählten Behandlungsbereichen (14)

2. Verfahren nach Anspruch 1, **dadurch gekennzeichnet, dass** die Behandlungsdauer des Behandlungsbereiches (14) und der Abstand der Strahlungsquelle zur Behandlungsfläche in Abhängigkeit von der Strahlungsleistung so gewählt wird, dass keine Schädigung der Haut eintritt.

3. Vorrichtung zur Durchführung einer selektiven Hyperthermie mittels elektromagnetischer Wellen zur Behandlung des menschlichen oder tierischen Körpers, **dadurch gekennzeichnet, dass** die zu behandelnde Person (12) auf einer Liege (1) liegt, oder das zu behandelnde Tier auf einer Behandlungsebene fixiert ist, über der im Abstand eine Laufschiene (3) angeordnet ist, in der ein Schlitten (4) verfahrbar ist, der ein oder mehrere IR-Strahlungsquellen (7) trägt, die gegen den menschlichen oder tierischen Körper gerichtet sind.

4. Vorrichtung nach Anspruch 3, **dadurch gekennzeichnet, dass** der Schlitten (4) in Richtung der Längsachse (13) verfahrbar angetrieben ist.

5. Vorrichtung nach Anspruch 3 oder 4, **dadurch gekennzeichnet, dass** der Schlitten (4) in Richtung der Querachse verfahrbar angetrieben ist.

6. Vorrichtung nach einem der Ansprüche 3 bis 5, **dadurch gekennzeichnet, dass** der vertikale Abstand der IR-Strahlungsquelle (7) zur Liege (1) einstellbar ist.

7. Vorrichtung nach einem der Ansprüche 3 bis 6, **dadurch gekennzeichnet, dass** der Schlitten (4) oszillierend in der X-Y-Ebene verschiebbar angetrieben ist.

8. Vorrichtung nach einem der Ansprüche 3 bis 7, **dadurch gekennzeichnet, dass** der Schlitten (4) oszillierend in der Z-Achse verschiebbar angetrieben ist.

9. Vorrichtung nach einem der Ansprüche 3 bis 8, **dadurch gekennzeichnet, dass** der Schlitten (4) oszillierend verschiebbar in der X-Y-Ebene und zusätzlich in der Z-Achse verschiebbar angetrieben ist.

10. Vorrichtung nach einem oder mehreren der Ansprüche 3 bis 9, **dadurch gekennzeichnet, dass** sie zur Durchführung des Verfahrens nach einem der Ansprüche 1 bis 2 geeignet ist.
